# EUROPEAN PATENT SPECIFICATION

(11) **EP 3 713 612 B1**
(45) Date of publication and mention of the grant of the patent: **19.06.2024**
(21) Application number: 17823209.6
(22) Date of filing: 13.11.2017
(51) Int. Cl.: F24F 8/117, F24F 8/133, F24F 3/16, A61L 9/14, B01D 47/06

(54) **AIR PURIFIER APPARATUS**
LUFTREINIGUNGSVORRICHTUNG
APPAREIL PURIFICATEUR D'AIR

(43) Date of publication of application: 30.09.2020
(73) Proprietor: Solidea S.r.l., 41051 Castelnuovo Rangone (MO) (IT)
(72) Inventor: SOLA, Enzo, 41028 Serramazzoni (MO) (IT); VENTURI, Marco, 41051 Castelnuovo Rangone (MO) (IT); MONTORSI, Igor, 41051 Castelnuovo Rangone (MO) (IT); BENEDETTI, Gianluca, 41051 Castelnuovo Rangone (MO) (IT)
(74) Representative: Brunacci, Marco
(86) International application number: PCT/IB2017/057077
(87) International publication number: WO 2019/092485

(56) References cited:
- EP-A2- 0 301 212
- US-A- 6 059 866
- US-A1- 2005 072 308
- US-A1- 2005 109 209

## Description

The invention relates to an air purifier apparatus, in particular for environments inside buildings, of the portable type.

The pollutants that are detected in very crowded buildings and workplaces, such as offices and/or shopping malls, are of a heterogeneous nature - for example organic volatile compounds, mineral fibres, microorganisms in the form of fungi and bacteria, carbon oxide, etc - and are present in high concentrations through the effect of the continuous activities that are performed there.

These pollutants are the cause of numerous infections and disorders such as headaches, excessive tiredness, mucous membrane irritation, colds, influenza and allergies that have significant repercussions on the health of millions of people.

The conditioning systems themselves, if they are present in crowded environments such as working and/or commercial buildings, if they are not subject to periodic maintenance and cleaning, can constitute sources of proliferation of pathogenic germs such as for example moulds, fungi, viruses and bacteria that are the cause of allergies, unwellness, and headaches in the persons who occupy the environments.

The increasingly frequent use in work environments of air conditioning systems creates the need to safeguard healthiness of the air that is generated by the air conditioning systems. There is in fact a close relationship between the quality of the air in the buildings and the health of the persons occupying the buildings.

Portable air purifier apparatuses are known that use filters, such as active carbon filters, or HEPA filters to retain the powder present in the air. Nevertheless, these apparatuses show a significant reduction in dust reduction efficiency during the life thereof owing to the filters that collect dust and dirt. In such conditions, a part of the very fine dust particles is not retained and is again recirculated, thus reducing the effect of the purifier. When the filters are clogged, they have to be replaced, with consequent expenditure of time and financial resources.

Air purifier apparatuses are known that purify the air by a liquid, in particular by water, consisting of a housing provided with an inlet from which the air to be purified enters and with an outlet for the purified air. The air to be purified enters the housing through a suction valve arranged for sucking the air to be purified from the environment outside the air purifier apparatus.

The water is distributed by a spray nozzle inside the housing in the form of drops that meet the flow of air exiting the aspirator.

Solid particles present in the air are captured by the drops of washing liquid by contact.

Apparatuses of known type provide a vertical extension of the housing, such that the drops of water fall by gravity, loading up with solid particles, to a bottom zone of the housing.

An air purifier of this type is disclosed in DE20312576 Ul, in which a spray nozzle is positioned inside the housing so as to generate a flow of drops of water that is in a current with the air flow to be purified coming from the aspirator.

In order to increase removal of the particulate, in some apparatuses the air flow to be purified is pushed against the current by the flow of drops of water as in the apparatus disclosed in WO200607225 A2.

However, in the known types of apparatus indicated above, the spray of water generated by the spray nozzle is not able to reach each zone of the housing. In particular, the spray of water produced by the spray nozzle has a greater density of drops near the spray nozzle whereas the spray of water has a lesser density of drops near the inner walls of the housing. This means that the air that traverses the housing near the inner walls of the housing is purified only partially, as the number of contacts between the drops and the air in this zone is limited.

One drawback of the air purifier apparatuses of known type is that the efficacy of the washing of the air is not optimum.

IT1401556 discloses an air purifier apparatus that is extremely effective in which the drawbacks disclosed above are resolved by using an air housing or washing chamber of the air that extends in a horizontal direction.

The air exiting the housing contains a much lower percentage of humidity than what occurs in apparatuses with a vertical housing, nevertheless, the need is felt to improve the control of the humidity exiting the housing.

US 6059866 A discloses an air washer having a basic unit comprising a water spray chamber in which an air flow takes place from an air inlet to an air outlet; an inlet washer media disposed in the air inlet of the water spray chamber; an outlet washer media disposed in the air outlet of the water spray chamber; first stage nozzles from which spraying water that reaches the inlet washer media is sprayed in the reverse direction to the air flow, located downstream from the inlet washer media; a water storage tank that receives spraying water running down, located below the water spray chamber; and a circulating water supply system that circulates the circulating water in the water storage tank to the first stage nozzles. There are also provided second stage nozzles from which spraying water directed against the outlet washer media is sprayed in the forward direction to the air flow, disposed at approximately the same positions as the first stage nozzles; and a supplementary water supply system for supplying supplementary water to the second stage nozzles.

US2005/0072308 A1 discloses an air cleaner for cleaning air and for providing sterilization and deodorization effects, which includes an air-liquid contact part, for bringing the air into contact with a water screen formed by spraying, using nozzles, pressurized circulating water, and for removing foreign materials from the air; a liquid separating part for separating from the air, tiny water droplets that are included in the air passing through the air-liquid contact part; a cylindrical body into which air to be cleaned is guided, and in which the air-liquid contact part and the liquid separating part are arranged in order; a chlorine dioxide addition unit, for adding a stabilized chlorine dioxide solution to a water tank for the water that is circulated; and a chlorine dioxide activation unit, for activating chlorine dioxide added to the circulating water, that is arranged in a circulating water pipe that extends from the water tank to the spray nozzles.

EP 0301212 A2 discloses an air humidifier comprising a flow duct, in which there is arranged a water spray nozzle which is active counter to the direction of flow of the air to be humidified. In front of its nozzle opening, there is a deflection surface which serves for the radial atomisation, at least approximately over the entire duct cross-section, of the water jet coming out of the nozzle opening. As a result, a very fine atomisation of the water can be achieved transversely to the direction of flow of the air to be humidified, which is thus passed through by very fine droplets which, under the action of the energy content of the air flowing through the flow duct, evaporate, which results in an adiabatic air humidification.

One of the objects of the present invention is to improve known air purifier apparatuses and, in particular, to meet the above need to control the humidity of the air exiting the purifying apparatus.

A further object is thus to provide an air purifier apparatus that can improve the condition of humidity of the environment in which it is inserted.

Still another object is to increase the efficacy of elimination of contaminants present in the air to be purified.

A further object of the invention is to obtain a versatile purifying apparatus, which can be used in environments inside buildings.

According to the invention, an air purifier apparatus is provided, as defined in claim 1.

The invention can be better understood and implemented with reference to the attached drawings, which illustrate some embodiments thereof by way of non-limiting example, in which:
Figure 1 is a frontal perspective view of an air purifier apparatus;
Figure 2 is a view of the air purifier apparatus of Figure 1, from which a front and side part of the guard and a lid have been removed;
Figure 3 is a lateral view of the air purifier apparatus of Figure 1, devoid of a guard and lid;
Figure 4 is a schematic section view of the air purifier apparatus of Figure 1;
Figure 5 is a perspective view of a washing chamber of the air purifier apparatus of Figure 1 placed against further washing chambers that are the same as the washing chamber;
Figure 6 shows a front view with liquid dispensing means of the air purifier apparatus;
Figure 7 is a schematic longitudinal section of the liquid dispensing means of Figure 6;
Figure 8 is a lateral view of the liquid dispensing means in Figure 6.

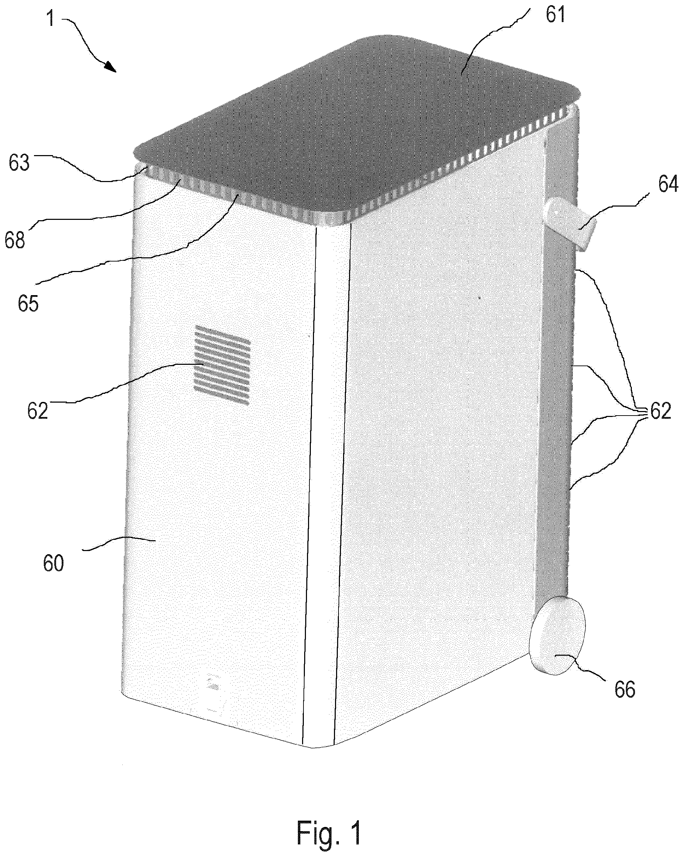

Figures 1-4 show an air purifier apparatus 1 for purifying air, in particular for environments inside buildings, of the portable type. As will be disclosed below, the air purifier apparatus 1 comprises condensation regulating means that provides the advantage of having greater control of the humidity of the air exiting the apparatus 1 than with prior-art apparatuses.

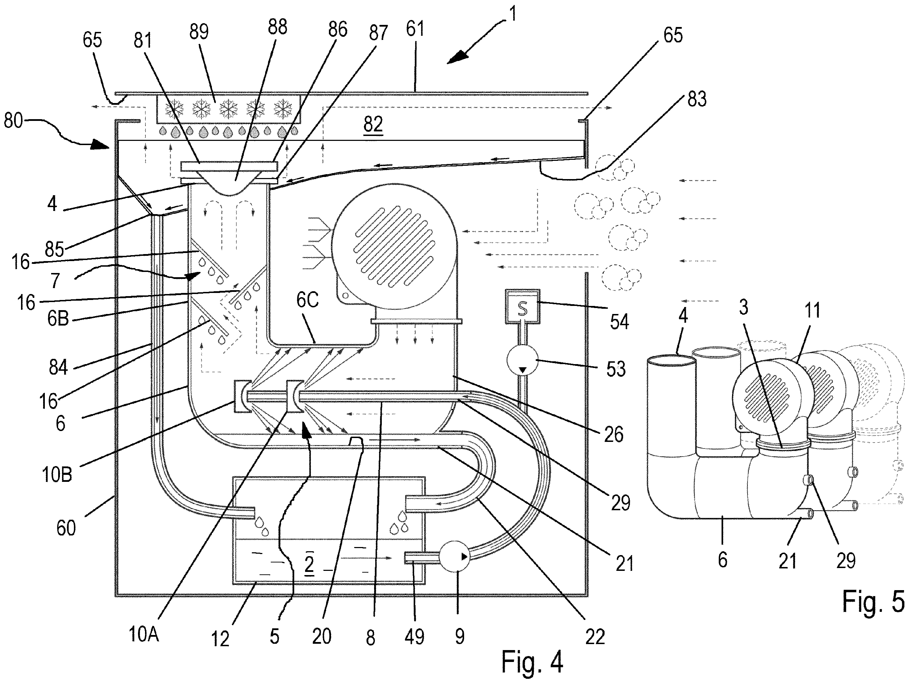

The air purifier apparatus 1 purifies air by a liquid 2 and comprises a body 6, shown isolated in Figure 5, in which a washing chamber for the air is obtained. The body 6 is provided with an inlet port or inlet 3 from which air to be purified enters and with an outlet port or outlet 4 from which air exits that has been purified, in particular purified of solid particles, such as dust, pollen, and further of bacteria and of oils in suspension.

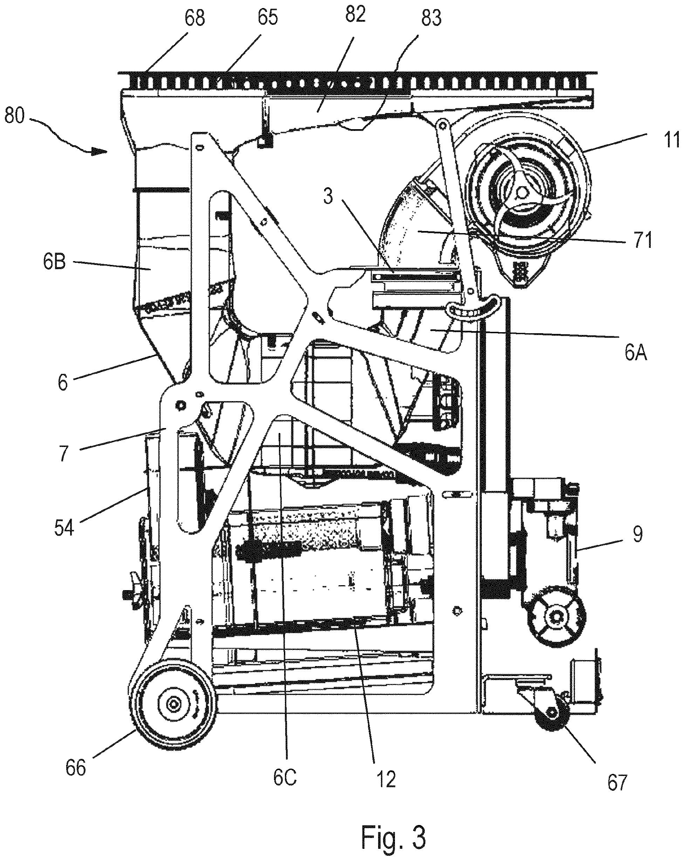

The body 6 is intended for being traversed between the inlet 3 and the outlet 4 by an air flow generated by air flow generating means, such as for example a fan 11. The fan 11 is mounted outside the body 6, for example at the inlet 3, as shown in Figure 4, or with the interposition of a manifold 71, as shown in Figure 3.

The body 6 comprises a first portion or inlet portion 6A in which the inlet 3 is obtained, a second portion or outlet portion 6B in which the outlet 4 is obtained. Between the first portion 6A and the second portion 6B an intermediate portion or washing chamber 6C is interposed, in which the air to be purified comes into direct contact with the liquid 2 to be washed, as will be disclosed better below.

The air purifier apparatus 1 comprises a casing or guard 60 that surrounds the inside of the apparatus and an upper closing lid 61. In a frontal region and/or in a rear region of the guard 60 inlet 62 fissures are obtained for the entry of the air into the apparatus. Naturally, the number of inlet 62 slits can be greater than that shown and the arrangement of the inlet 62 slits can affect a more extensive region of the guard 60 than that illustrated. Between the guard 60 and the lid 61 an outlet region 63 is defined for the exiting of the air treated by the air purifier apparatus 1. The outlet region 63 comprises a bounding wall or cornice 68 that is provided with a plurality of openings 65; the arrangement of the openings 65 extends along the entire perimeter of the cornice 68 or can extend only along one or more sides thereof.

The air purifier apparatus 1 comprises a frame 70 that supports the various parts of the apparatus. The frame 70 is provided with a pair of rear wheels 66, and optionally with a front wheel 67, which enable the user to move the apparatus and arrange the apparatus in a preferred zone inside the environment to be purified. A handle 64, for example fixed to a rear part of the apparatus, can be grasped by the user to move the apparatus.

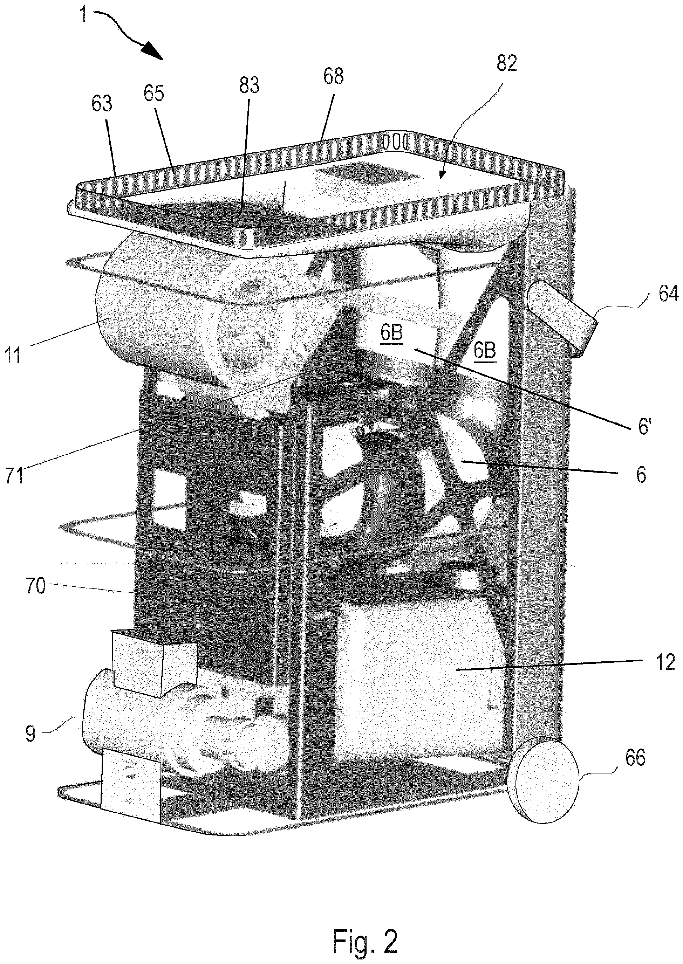

With reference to Figure 5, the body 6 is configured for washing a preset amount of air and defines a module, also known as a washing module, that can be mounted next to further identical modules to produce an apparatus that is able to process an air flow increased by an amount corresponding to the number of modules mounted together. In the embodiment of the air purifier apparatus 1 illustrated in Figures 1-4, there are two washing modules, i.e. the body 6 and the body 6' that are the same as one another, shown in Figure 2. In this case, as in the cases of a number of bodies 6 greater than two, the respective inputs 3 can receive air from a manifold 71 and be supplied by the same fan 11 fitted on the manifold 71, instead of having a fan for each inlet as illustrated in the embodiment of Figure 5.

Still with reference to Figure 4, the body 6 comprises liquid dispensing means 5 arranged inside the washing chamber 6C to dispense the liquid 2 in the form of drops.

The air to be purified is then removed from an environment outside the body 6 and the apparatus 1, such as for example a domestic environment, or an environment inside a building, and is conveyed by the fan 11 to the first portion 6A, where the air changes motion direction and reaches the washing chamber 6C. The washing chamber 6C extends along a direction of longitudinal extent that is substantially horizontal. "Substantially horizontal" is understood to be a direction that can deviate from a horizontal axis of a Cartesian reference system - i.e. an axis perpendicular to the gravity acceleration vector-also by 5 degrees. The washing chamber 6C has a tubular circular section shape.

Although the path of the air inside the washing chamber 6C is not linear, the air advances inside the washing chamber 6C along an advancement or main direction D. The main direction D of the air flow inside the washing chamber 6C is determined by the inner shape of the washing chamber 6C and is thus parallel to the longitudinal extent direction, which is also substantially horizontal.

The first portion 6A and the second portion 6B are arranged at opposite ends of the washing chamber 6C such that the body 6, and thus the path travelled by the air inside the housing 6, substantially has the shape of a "U" - although one arm of the "U", the arm of the inlet of the air, is shorter than the other arm, the arm of the outlet of the air.

The liquid 2 used in the air purifier apparatus 1 to purify the air inside a building is for example water. The liquid 2 can comprise, in addition to the water, one or more substances, such as for example, an antibacterial substance S to lower bacteria found in the air to be purified. The antibacterial substance S is contained in a rechargeable container 54 and is sent automatically to the liquid dispensing means 5 by a pumping device 53 of known type. In this manner, the liquid 5 that washes the air acquires an antibacterial power, sanitizing the air of the environment in which the air purifier apparatus 1 is housed.

The liquid 2 is contained in a tank 12, arranged outside the body 6.

The liquid 2 is removed from the tank 12 by a pump 9, which is also arranged outside the body 6. By a delivery conduit 49, shown in Figure 4, having an open end inside the tank 12 and immersed in the liquid 2, the pump 9 sends the liquid 2 to the liquid dispensing means 5 in the washing chamber 6C.

Through the pump 9, the liquid 2 is removed from the tank 12, is brought to a desired pressure value and is then delivered to a supply conduit 8 supplying the liquid dispensing means 5.

The conduit 8 traverses or is connected to a hole 29 obtained in an end wall 26 of the first portion 6A. On this end wall 26, at a bottom zone 20 of the washing chamber 6C a hole 21 is further provided that is connected to a pipe 22 that connects the washing chamber 6C to the tank 12, returning the water that has collected in the body 6 to the tank 12.

Again with reference to Figure 4, the air flow that has interacted with the liquid 2 enters the second portion 6B of the body 6. The second portion 6B, unlike the washing chamber 6C, extends prevalently in a vertical direction. The second portion 6B forms as it were an exit flue for the purified air.

As the illustrated embodiments comprise two bodies 6, 6', two second portions are present 6B, which are alongside one another.

Each second portion 6B comprises liquid condensing means 7, arranged for capturing drops of the liquid 2 which are possibly conveyed from the air flow downstream of the dispensing means 5.

The liquid condensing means 7 comprises a plurality of deflecting elements 16 arranged so as to define a labyrinth path for the air flow that traverses the second portion 6B. Each deflecting element 16 comprises for example a laminar element, such as in particular a metal sheet, for example drilled metal sheet or a grille. When the flow of air that traverses the second portion 6B comes into contact with the deflecting elements 16, the liquid 2 possibly present in the flow of air condenses against the deflecting elements 16, which act as drop separators. The drops fall inside the washing chamber 6C and collect in the bottom zone 20 thereof.

In the embodiment of Figure 4 three deflecting elements are provided, but naturally the number of deflecting elements 16 can be different, for example greater or less than three. In Figure 4, the deflecting elements 16 are represented as flat portions that protrude and tilt downwards, i.e. to the bottom 20 of the washing chamber 6C, fixed alternatively to an inner wall of the second portion 6B. Alternatively or additionally, each deflecting element 16 can have the shape of a disc fixed circumferally to the inner wall of the second portion 6B and have a cavity structure, to capture by compensation also the finer drops of liquid 2. The air purifier 1 apparatus further comprises condensation regulating means 80, arranged for increasing the condensation of the humidity contained in the air present in the second portion 6B and downstream of the second portion 6B, so as to reduce the humidity of the air exiting said apparatus 1.

The condensation regulating means 80 comprises an expansion chamber 82 to enable the air exiting the second portion 6B to expand before exiting the apparatus 1.

The expansion chamber 82 is connected to the second portion 6B to receive air coming therefrom and extends above the outlet port 4. The expansion chamber 82 comprises a tank 83 for collecting the condensation, which is bounded above by the lid 61.

Between the tank 83 and the lid 61 there is the bounding wall or cornice 68 disclosed above, provided with the plurality of openings 65 for the exit of the air from the expansion chamber 82 and, thus, from the apparatus 1.

As Figure 4 shows, a base wall of the tank 83 is provided with a hole 85 for conveying the water, condensed on the walls of the expansion chamber 82, to a pipe 84 connected to the tank 12.

Owing to the expansion chamber 82, control of the conveyed water content of the flow of air exiting the second portion 6B is promoted because owing to the expansion of the air the quantity of water that condenses on the walls is increased.

The condensation regulating means 80 further comprises a closure element 81 arranged at the outlet port 4 to close the outlet port and create an overpressure of the air inside the second portion 6B. In this manner, the steam tension of the water conveyed by the air inside the outlet portion 6B is reduced.

The closure element 81 is movable away from, and towards, the outlet port 4 on the basis of the pressure of the air present in the second portion 6B.

When the pressure of the air present in the second portion 6B exceeds the sum between the pressure in the expansion chamber 82 and the pressure corresponding to the mass of the closure element 81, the latter is moved away from the outlet 4 and lifted therefrom, creating a narrowing that increases the speed of the air that is directed to the expansion chamber 82. On the other hand, if the pressure in the second portion 6B is not sufficient, the closure element 81 remains in the position nearer the outlet 4, acting as a closing cap.

The closure element 81 comprises a disc member 86 resting on an abutting surface 87 of the outlet port 4. The disc member 86 is provided with a protruberance 88 shaped for engaging a region of the outlet port 4 inside the second portion 6B.

The disc member 86 naturally has a calibrated mass that enables the apparatus 1 to operate with a slight overpressure inside the body 6, which promotes the depositing of drops of water onto the inner walls of the second portion 6B and/or onto the liquid condensing means 7, in particular onto the deflecting elements 16.

The closure element 81, together with the expansion chamber 82, contributes to controlling the humidity of the air of the environment into which the air purifier apparatus 1 is inserted. It has been ascertained by experiment that the air purifier apparatus 1 that is thus structured reduces the humidity of the air of environments that are too humid and increases the humidity of the air of environments that are excessively dry to reach a humidity level considered to correspond to the state of wellbeing of persons.

The condensation regulating means 80 can further comprise a cooling device 89 facing the outlet port 4 to cool the expanding air in the expansion chamber 82 and further promote the reduction of the humidity exiting the apparatus 1. The cooling device 89 can be driven or can be overridden by operation by the user of a control panel provided in the apparatus 1.

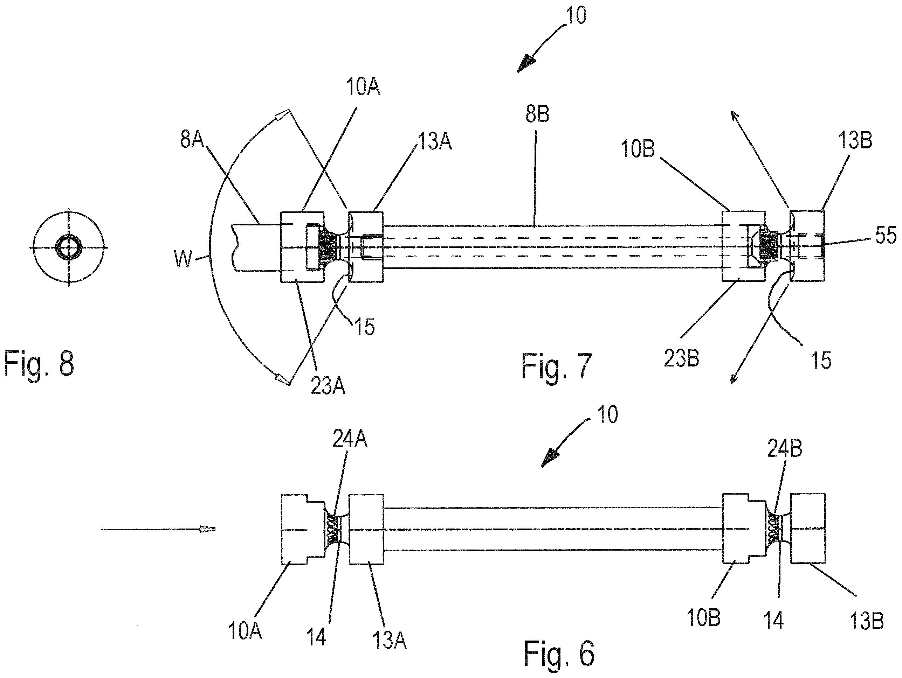

With reference now to Figures 6 and 7, the liquid dispensing means 5 comprises a plurality of nozzles 10, for example two nozzles 10A and 10B. In particular, the nozzles 10A and 10B are structurally and functionally the same as one another. The nozzles 10A and 10B are shaped to create a flow of drops of water that are substantially against the current of the flow of air that traverses the body 6.

Each nozzle 10A, 10B of the plurality of nozzles is connected to the pump 9 by the same supply conduit 8.

The conduit 8 comprises a first portion 8A that extends horizontally inside the washing chamber 6C, i.e. along a direction that is substantially parallel to the longitudinal extent direction of the washing chamber 6C and supplies the first nozzle 10A. The conduit 8 further comprises a second portion 8B, which is also substantially horizontal, that supplies the second nozzle 10B, fitted to the free end of the conduit 8. The nozzles 10A, 10B are thus arranged in succession along the same conduit 8 such that the first nozzle 10A is mounted interposed between the first portion 8A and the second portion 8B of the conduit 8.

Compared with nozzles that are traditionally to be found in air purifier apparatuses of known type, the nozzles 10A, 10B are shaped to emit drops of water according to a cone having a symmetry axis coinciding with the axis 8 of the supply, i.e. horizontal, conduit. On the other hand, in known apparatuses, the emission cone is generally orthogonal to the axis of the supply conduit.

The nozzles 10A, 10B are designed to enable drops of water to be produced having a dimension that is similar to that of the aforesaid "fine dusts", i.e. significantly increasing the effect according to which the drops of water succeed in capturing or entrapping the dust particles.

This is made possible by the particular conformation of the nozzles 10A, 10B.

The vertex angle W of the dispensing cone, when projected onto a plane is for example 120°. In other words, in this embodiment, the drops of water exiting nozzle means 10A, 10B, have a motion direction that forms with an axis of said nozzle means 10A, 10B an angle of 60°.

Each nozzle 10A, 10B is provided with a respective union member 23A 23B, which is hollow to permit the transit of the water and is provided internally with a thread for fitting the nozzle 10A, 10B to the respective sections of the conduit 8. Each nozzle 10A, 10B then comprises an arm 24A, 24B on which dispensing holes 14, in particular calibrated dispensing hole 14, are obtained. For example, five outlet holes can be present that are angularly equidistant on the respective arm 24A, 24B.

Each nozzle 10A, 10B then comprises a diffuser element 13A, 13B. The diffuser element 13A, 13B comprises a concave surface 15 having concavity facing the arm 24A, 24B of the nozzle 10A, 10B.

The concave surface 15 is for example a portion of spherical surface. The concave surface 15 is shaped so as to break a jet of the liquid 2 coming from the nozzle 10A, 10B and divide the jet into a spray of drops. The diffuser element 13A comprises a through hole for transferring the water coming from the first section 8A to the second section 8B of the conduit 8. The diffuser element 13B thus has an end cap 55 that does not permit a further exit of the water.

The same structure for the two nozzles permits easy production and replacement thereof.

The pressurized liquid 2, exiting the nozzle 10A, 10B, hits the diffuser element 13A, 13B and reverses the direction of the axial speed component thereof.

The liquid dispensing means 5 is shaped so as to generate drops, each drop having a speed component opposite the main direction D of air flow.

The diffuser element 13A, 13B produces drops indirectly, i.e. by reflection of the jet of the liquid 2 coming from the nozzle 10A, 10B divided by the dispensing holes 14 against the concave surface 15. The generated drops have a sufficiently small dimension to capture the fine powders but sufficiently high dimensions to fall into the bottom zone 20 of the body 6, preventing the drops being transported by the air flow that traverses the washing chamber 6C.

The liquid 2 is thus supplied in the form of rain or spray and moves with a larger "horizontal" speed component inside the washing chamber 6C through the liquid dispensing means 5. In this manner, the drops meet the air flow to be purified at least partially against the current.

Against the current, a higher number of contacts occurs between the drops and the air to be purified. In this manner, the purifying capacity of the liquid 2 is optimized. Further, the horizontal component of the speed of the drops and the horizontal direction of the air flow enable excessive humidification of the air during washing to be limited.

Further, the horizontal arrangement of the washing chamber 6C enables the air to spend a longer time in the washing chamber 6C than apparatuses of known type that have a vertical housing, this permitting more effective purification of the air.

The successive arrangement of the nozzles 10A, 10B along the longitudinal extent direction of the washing chamber 6C enables a plurality of washing fronts to be created in the main advancement direction D of the flow of air. In this manner, possible polluting substances still present in the air that has been partially purified by a first washing front can be captured by the further washing front that the air meets in the horizontal path inside the washing chamber 6C to the exit 4.

The liquid dispensing means 5 is arranged such that a geometric axis of each nozzle 10A, 10B is substantially parallel to the longitudinal extent direction of the washing chamber 6C.

The liquid dispensing means 5 is moreover coaxial.

The contact between the liquid 2 in the form of drops and the air flow inside the washing chamber 6C enables the polluting particles to be captured that are present in the air to be purified.

From experiments conducted using the purifying apparatus 1, it has emerged that by collecting the particulate of the air of the environment before and after operation of the apparatus 1 for a certain time, corresponding to a standardized volume of air of 3.6Nm³ at 25°C, reduction of the dust in the air of the environment is complete, i.e. is 100%.

The liquid 2 coming from the nozzles 10, the liquid condensed on the deflecting elements 16 - and on the liquid-capturing element if present - and the solid particles "precipitated" and captured by the liquid 2, fall by gravity and are collected in the bottom zone 20 of the body 6. The liquid 2 is transferred from the bottom zone 20 inside the tank 12 by the pipe 21.

Once inside the tank 12, the polluting particles are deposited on the bottom of the tank 12 by sedimentation.

The washing machine 2 can be changed or topped up in the tank 12 through an opening provided in a part of the tank 12 and not illustrated. An automatic seal valve, which is not shown, enables the tank 12 to be removed and refitted after filling without it being necessary to use particular tools.

The shape of the air purifier apparatus 1 enables the liquid 2 to be used for multiple purification cycles, thus limiting the consumption thereof.

The quantity of liquid 2 necessary for each purification cycle is moreover rather reduced.

The purifying apparatus 1 further comprises a control unit provided with the control unit for selecting the speed of the fan, the duration of the operation of the apparatus 1 and alerting the user when the level of water in the tank 12 and/or of the antibacterial substance has fallen below the respective preset threshold values.

The air flow that interacts with the liquid 2, would have a humidity level that is much higher than the humidity of the flow of air entering from inlet 3 taken from the environment but less than the humidity reached by air in apparatuses of known type if the condensation regulating means 80 was not present.

Owing to the condensation regulating means 80, it is possible to limit the quantity of humidity exiting the air purifier apparatus and thus adjust the humidity of the environment the air of which is being purified.

## Claims

1. An air purifier apparatus (1) of the portable type for purifying air inside a building by a liquid (2) comprising a body (6) provided with: an inlet portion (6A) and an outlet portion (6B) for the air; with an intermediate washing chamber (6C) between said inlet portion (6A) and said outlet portion (6B) and intended for being traversed by an air flow along a substantially horizontal main advancement direction (D), said washing chamber (6C) extends along a direction of longitudinal extent that is substantially horizonal; and with liquid dispensing means (5) arranged inside said washing chamber (6C) to dispense said liquid (2) in the form of drops at least partially counter current with said air flow, said air purifier apparatus (1) further comprising condensation regulating means (80) arranged for increasing the condensation of the humidity contained in the air in said outlet portion (6B) and downstream of said outlet portion (6B) so as to reduce the humidity of the air exiting said apparatus (1), said condensation regulating means (80) comprising an expansion chamber (82) to enable said air exiting said outlet portion (6B) to expand before exiting said apparatus (1) **characterized in that**:
- said expansion chamber (82) is connected to said outlet portion (6B) to receive air coming from said outlet portion (6B) and extends horizontally above an outlet port (4) of said outlet portion (6B);
- said outlet portion (6B), unlike said washing chamber (6C), extends prevalently in a vertical direction.

2. Air purifier apparatus (1) according to claim 1, wherein said expansion chamber (82) comprises a tank (83) for collecting the condensate, said tank (83) being bounded above by a lid (61).

3. Air purifier apparatus (1) according to claim 2, wherein between said tank (83) and said lid (61) a bounding wall (68) is present that is provided with a plurality of openings (65) for the exit of said air from said expansion chamber (82) and thus from said apparatus (1).

4. Air purifier apparatus (1) according to claim 2, or 3, wherein a base wall of said tank (83) is provided with a hole (85) for conveying the condensed water to a pipe (84) connected to a reservoir (12) arranged for containing said liquid (2).

5. Air purifier apparatus (1) according to any preceding claim, wherein said condensation regulating means (80) further comprises a closing element (81) arranged at said outlet port (4) of said outlet portion (6B) to close said outlet port (4) to create air overpressure inside said outlet portion (6B) and reduce the vapour pressure of the water conveyed by said air inside said outlet portion (6B).

6. Air purifier apparatus (1) according to claim 5, wherein said closure element (81) is movable away from, and towards, said outlet port (4) on the basis of air pressure present in said outlet portion (6B).

7. Air purifier apparatus (1) according to claim 6, wherein said closure element (81) comprises a disc member (86) resting on an abutting surface (87) of said outlet port (4),

8. Air purifier apparatus (1) according to claim 7, wherein said disc member (86) is provided with a protuberance (88) shaped to engage a region of said outlet port (4) inside said outlet portion (6B).

9. Air purifier apparatus (1) according to any preceding claim, wherein said condensation regulating means (80) further comprises a cooling device (89) facing said outlet port (4) to cool said air exiting said outlet port (4).

10. Air purifier apparatus (1) according to any preceding claim, wherein said liquid dispensing means (5) comprises nozzle means (10A, 10B) provided with a diffuser element (13A, 13B), for dividing a jet of said liquid (2) exiting said nozzle means (10A, 10B) and forming drops of water, in which said diffuser element (13A, 13B) is so shaped that said drops of water exiting said nozzle means (10A, 10B) have a motion direction that forms a dispensing cone with a symmetry axis coinciding with a conduit axis of a supply conduit (8) of said nozzle.

11. Air purifier apparatus (1) according to claim 10, wherein said dispensing cone has a plan-view vertex angle (W) of 60°.

12. Air purifier apparatus (1) according to claim 10, or 11, wherein said diffuser element (13A, 13B), comprises a concave surface (15) facing an opening (14) of said nozzle means (10A, 10B).

## Patentansprüche

1. Tragbare Luftreinigungsvorrichtung (1) zum Reinigen von Luft im Inneren eines Gebäudes durch eine Flüssigkeit (2), umfassend einen Körper (6), der versehen ist mit: einem Einlassabschnitt (6A) und einem Auslassabschnitt (6B) für die Luft; mit einer Zwischen-Waschkammer (6C) zwischen dem Einlassabschnitt (6A) und dem Auslassabschnitt (6B), die dazu bestimmt ist, von einem Luftstrom entlang einer im Wesentlichen horizontalen Hauptbewegungsrichtung (D) durchströmt zu werden; wobei sich die Waschkammer (6C) entlang einer im Wesentlichen horizontalen Längsrichtung erstreckt; und mit Flüssigkeitsabgabemitteln (5), die innerhalb der Waschkammer (6C) angeordnet ist, um die Flüssigkeit (2) in Form von Tropfen zumindest teilweise im Gegenstrom zu dem Luftstrom abzugeben, wobei die Luftreinigungsvorrichtung (1) ferner Kondensationsregulierungsmittel (80) umfasst, die angeordnet sind, um die Kondensation der in der Luft enthaltenen Feuchtigkeit in dem Auslassabschnitt (6B) und stromabwärts des Auslassabschnitts (6B) zu erhöhen, um die Feuchtigkeit der die Vorrichtung (1) verlassenden Luft zu verringern, wobei die Kondensationsregulierungsmittel (80) eine Ausdehnungskammer (82) umfassen, um es der aus dem Auslassabschnitt (6B) austretenden Luft zu ermöglichen, sich auszudehnen, bevor sie die Vorrichtung (1) verlässt, **dadurch gekennzeichnet, dass**:
- die Ausdehnungskammer (82) mit dem Auslassabschnitt (6B) verbunden ist, um von dem Auslassabschnitt (6B) kommende Luft aufzunehmen, und sich horizontal oberhalb einer Auslassöffnung (4) des Auslassabschnitts (6B) erstreckt;
- der Auslassabschnitt (6B) sich anders als die Waschkammer (6C) überwiegend in vertikaler Richtung erstreckt.

2. Luftreinigungsvorrichtung (1) nach Anspruch 1, wobei die Ausdehnungskammer (82) einen Tank (83) zum Sammeln des Kondensats umfasst, wobei der Tank (83) oben durch einen Deckel (61) begrenzt ist.

3. Luftreinigungsvorrichtung (1) nach Anspruch 2, wobei zwischen dem Tank (83) und dem Deckel (61) eine Begrenzungswand (68) vorhanden ist, die mit einer Vielzahl von Öffnungen (65) für den Austritt der Luft aus der Ausdehnungskammer (82) und somit aus der Vorrichtung (1) versehen ist.

4. Luftreinigungsvorrichtung (1) nach Anspruch 2 oder 3, wobei eine Bodenwand des Tanks (83) mit einer Öffnung (85) versehen ist, um das kondensierte Wasser zu einem Rohr (84) zu leiten, das mit einem Behälter (12) verbunden ist, der die Flüssigkeit (2) enthält.

5. Luftreinigungsvorrichtung (1) nach einem der vorhergehenden Ansprüche, wobei die Kondensationsregulierungsmittel (80) ferner ein Verschlusselement (81) umfassen, das an der Auslassöffnung (4) des Auslassabschnitts (6B) angeordnet ist, um die Auslassöffnung (4) zu verschließen, um einen Luft-Überdruck innerhalb des Auslassabschnitts (6B) zu erzeugen und den Dampfdruck des von der Luft in dem Auslassabschnitt (6B) beförderten Wassers zu verringern.

6. Luftreinigungsvorrichtung (1) nach Anspruch 5, wobei das Verschlusselement (81) auf der Grundlage des in dem Auslassabschnitt (6B) vorhandenen Luftdrucks von der Auslassöffnung (4) weg und zu ihr hin bewegbar ist.

7. Luftreinigungsvorrichtung (1) nach Anspruch 6, wobei das Verschlusselement (81) ein Scheibenelement (86) umfasst, das auf einer Anlagefläche (87) der Auslassöffnung (4) aufliegt.

8. Luftreinigungsvorrichtung (1) nach Anspruch 7, wobei das Scheibenelement (86) mit einer Ausstülpung (88) versehen ist, die so geformt ist, dass sie in einen Bereich der Auslassöffnung (4) innerhalb des Auslassabschnitts (6B) eingreift.

9. Luftreinigungsvorrichtung (1) nach einem der vorhergehenden Ansprüche, wobei die Kondensationsregulierungsmittel (80) ferner eine Kühlvorrichtung (89) umfassen, die der Auslassöffnung (4) zugewandt ist, um die aus der Auslassöffnung (4) austretende Luft zu kühlen.

10. Luftreinigungsvorrichtung (1) nach einem der vorhergehenden Ansprüche, wobei die Flüssigkeitsabgabemittel (5) eine Düsenmittel (10A, 10B) umfassen, die mit einem Diffusorelement (13A, 13B) versehen sind, um einen aus den Düsenmitteln (10A, 10B) austretenden Strahl der Flüssigkeit (2) zu teilen und Wassertropfen zu bilden, wobei das Diffusorelement (13A, 13B) so geformt ist, dass die aus den Düsenmitteln (10A, 10B) austretenden Wassertropfen eine Bewegungsrichtung haben, die einen Abgabekegel mit einer Symmetrieachse bildet, die mit einer Leitungsachse einer Versorgungsleitung (8) der Düse zusammenfällt.

11. Luftreinigungsvorrichtung (1) nach Anspruch 10, wobei der Abgabekegel in Draufsicht einen Scheitelwinkel (W) von 60° aufweist.

12. Luftreinigungsvorrichtung (1) nach Anspruch 10 oder 11, wobei das Diffusorelement (13A, 13B) eine konkave Oberfläche (15) aufweist, die einer Öffnung (14) der Düsenmittel (10A, 10B) zugewandt ist.

## Revendications

1. - Appareil purificateur d'air (1) du type portable pour purifier de l'air à l'intérieur d'un bâtiment par un liquide (2), comprenant un corps (6) comportant : une partie d'entrée (6A) et une partie de sortie (6B) pour l'air ; une chambre de lavage intermédiaire (6C) entre ladite partie d'entrée (6A) et ladite partie de sortie (6B) et destinée à être traversée par un écoulement d'air le long d'une direction d'avance principale sensiblement horizontale (D), ladite chambre de lavage (6C) s'étendant le long d'une direction d'extension longitudinale qui est sensiblement horizontale ; et un moyen de distribution de liquide (5) disposé à l'intérieur de ladite chambre de lavage (6C) pour distribuer ledit liquide (2) sous forme de gouttes au moins en partie à contre-courant dudit écoulement d'air, ledit appareil purificateur d'air (1) comprenant en outre un moyen de régulation de condensation (80) agencé pour augmenter la condensation de l'humidité contenue dans l'air dans ladite partie de sortie (6B) et en aval de ladite partie de sortie (6B) de façon à réduire l'humidité de l'air sortant dudit appareil (1), ledit moyen de régulation de condensation (80) comprenant une chambre de détente (82) pour permettre audit air sortant de ladite partie de sortie (6B) de se détendre avant de sortir dudit appareil (1), **caractérisé par le fait que** :
- ladite chambre de détente (82) est reliée à ladite partie de sortie (6B) pour recevoir de l'air provenant de ladite partie de sortie (6B) et s'étend horizontalement au-dessus d'un orifice de sortie (4) de ladite partie de sortie (6B) ;
- ladite partie de sortie (6B), contrairement à ladite chambre de lavage (6C), s'étend principalement dans une direction verticale.

2. - Appareil purificateur d'air (1) selon la revendication 1, dans lequel ladite chambre de détente (82) comprend un réservoir (83) pour collecter le condensat, ledit réservoir (83) étant délimité au-dessus par un couvercle (61).

3. - Appareil purificateur d'air (1) selon la revendication 2, dans lequel entre ledit réservoir (83) et ledit couvercle (61) se trouve une paroi de délimitation (68) qui comporte une pluralité d'ouvertures (65) pour la sortie dudit air de ladite chambre de détente (82) et donc dudit appareil (1).

4. - Appareil purificateur d'air (1) selon la revendication 2 ou 3, dans lequel une paroi de base dudit réservoir (83) comporte un trou (85) pour acheminer l'eau condensée jusqu'à un tuyau (84) relié à un réservoir (12) agencé pour contenir ledit liquide (2).

5. - Appareil purificateur d'air (1) selon l'une quelconque des revendications précédentes, dans lequel ledit moyen de régulation de condensation (80) comprend en outre un élément de fermeture (81) disposé à ledit orifice de sortie (4) de ladite partie de sortie (6B) pour fermer ledit orifice de sortie (4) de façon à créer une surpression d'air à l'intérieur de ladite partie de sortie (6B) et à réduire la pression de vapeur de l'eau transportée par ledit air à l'intérieur de ladite partie de sortie (6B).

6. - Appareil purificateur d'air (1) selon la revendication 5, dans lequel ledit élément de fermeture (81) est déplaçable à l'opposé de, et vers, ledit orifice de sortie (4) en fonction d'une pression d'air présente dans ladite partie de sortie (6B).

7. - Appareil purificateur d'air (1) selon la revendication 6, dans lequel ledit élément de fermeture (81) comprend un élément disque (86) reposant sur une surface d'appui (87) dudit orifice de sortie (4).

8. - Appareil purificateur d'air (1) selon la revendication 7, dans lequel ledit élément disque (86) comporte une protubérance (88) formée pour s'engager avec une région dudit orifice de sortie (4) à l'intérieur de ladite partie de sortie (6B).

9. - Appareil purificateur d'air (1) selon l'une quelconque des revendications précédentes, dans lequel ledit moyen de régulation de condensation (80) comprend en outre un dispositif de refroidissement (89) faisant face audit orifice de sortie (4) pour refroidir ledit air sortant dudit orifice de sortie (4).

10. - Appareil purificateur d'air (1) selon l'une quelconque des revendications précédentes, dans lequel ledit moyen de distribution de liquide (5) comprend un moyen de buse (10A, 10B) comportant un élément diffuseur (13A, 13B), pour diviser un jet dudit liquide (2) sortant dudit moyen de buse (10A, 10B) et former des gouttes d'eau, dans lequel ledit élément diffuseur (13A, 13B) est conformé de telle sorte que lesdites gouttes d'eau sortant dudit moyen de buse (10A, 10B) ont une direction de mouvement qui forme un cône de distribution ayant un axe de symétrie coïncidant avec un axe de conduit d'un conduit d'alimentation (8) de ladite buse.

11. - Appareil purificateur d'air (1) selon la revendication 10, dans lequel ledit cône de distribution a un angle au sommet (W) en vue en plan de 60°.

12. - Appareil purificateur d'air (1) selon la revendication 10 ou 11, dans lequel ledit élément diffuseur (13A, 13B) comprend une surface concave (15) faisant face à une ouverture (14) dudit moyen de buse (10A, 10B).
